(19) Europäisches Patentamt
European Patent Office
Office européen des brevets

(11) Publication number: 0 356 070 A1

# (12) EUROPEAN PATENT APPLICATION

(21) Application number: 89308121.6

(51) Int. Cl.4: C07D 301/14 , C07D 303/16

(22) Date of filing: 10.08.89

(30) Priority: 15.08.88 US 232412

(43) Date of publication of application: 28.02.90 Bulletin 90/09

(84) Designated Contracting States: AT BE CH DE ES FR GB GR IT LI LU NL SE

(71) Applicant: HOECHST CELANESE CORPORATION
Route 202-206 North
Somerville, N.J. 08876(US)

(72) Inventor: Aslam, Mohammad
7221 Diamond Ridge
Corpus Christi Texas(US)

(74) Representative: De Minvielle-Devaux, Ian Benedict Peter et al
CARPMAELS & RANSFORD 43, Bloomsbury Square
London WC1A 2RA(GB)

## (54) Synthesis of 4-acetoxystyrene oxide and related compounds.

(57) A method is provided of producing 4-acetoxystyrene oxide and substituted 4-acetoxystyrene oxides wherein at least one of the ring hydrogen atoms is substituted with a $C_1$ to $C_{10}$ alkyl or alkoxy, amino, halogen or nitro, wherein 4-acetoxystyrene or the corresponding substituted 4-acetoxystyrene is epoxided with peracetic acid containing no more than a trace of a mineral acid or water. The invention also includes, as new compositions of matter, 3,5-dimethyl-4-acetoxystyrene oxide and 3,5-dibromo-4-acetoxystyrene oxide.

EP 0 356 070 A1

# SYNTHESIS OF 4-ACETOXYSTYRENE OXIDE AND RELATED COMPOUNDS

## BACKGROUND OF THE INVENTION

### FIELD OF THE INVENTION

This invention relates to a new method of producing 4-acetoxystyrene oxide and related compounds, and to certain new substituted 4-acetoxystyrene oxides.

### BACKGROUND INFORMATION

It is known to utilize methylolated oxiranyl polymers of 4-acetoxystyrene oxide in the production of reverse osmosis membranes. Various substituted 4-acetoxystyrene oxides, as included in this disclosure, can also be used to form membranes with varying properties. 4-Acetoxystyrene oxide can also be used as an intermediate for the synthesis of certain pharmaceuticals, e.g., octopamine.

Also known is the production of 4-acetoxystyrene oxide by peroxidizing 4-acetoxystyrene with m-chloroperbenzoic acid. However, this peroxy compound is not readily available in commerce.

### DESCRIPTION OF RELATED ART

Japanese Application Publication (Kokai) No. Sho 58[1983]-92406 of Kawahara et al, published June 1, 1983, discloses the preparation of p-acetoxystyrene oxide by reacting p-acetoxystyrene with m-chloroperbenzoic acid dissolved in dichloromethane.

B. B. Corson et al, "Preparation of Vinylphenols and Isopropenylphenols," J. Org. Chem 23, 544-549, (April 1958), teach the preparation of p-vinylphenol from phenol in 53% yield by a five-step process: (1) acetylation of phenol to p-hydroxyacetophenone, (2) acetylation of the latter to p-acetoxyacetophenone, (3) hydrogenation of the ketone to p-acetoxyphenylmethylcarbinol, (4) dehydration of the carbinol to p-acetoxystyrene, (5) saponification of p-acetoxystyrene to p-vinylphenol.

G. G. Hawley, Condensed Chemical Dictionary, 10th ed., (Van Nostrand Reinhold, New York, 1981) page 786, discloses the use of peracetic acid for the "epoxidation of fatty acid esters and epoxy resin precursors."

D. J. Cram and G. S. Hammond, Organic Chemistry, (McGraw-Hill, New York, 1959), page 342, show the conversion of an alkene to an epoxide (oxirane) by oxidation with a percarboxylic acid such as peracetic acid, with the peracid being reduced to a carboxylic acid in the reaction. The authors state that "Since carboxylic acids can open oxide rings by nucleophilic substitution reactions, an insoluble weak base is often added to the reaction mixtures to neutralize the carboxylic acid."

Pending Application Serial No. 097,809, filed September 16, 1987, by Vicari et al discloses the production of 3,5-disubstituted-4-acetoxystyrene, e.g., 3,5-dimethyl-4-acetoxystyrene, by acetylating the corresponding 3,5-disubstituted-4-hydroxyacetophenone to obtain the 3,5-disubstituted-4-acetoxyacetophenone, hydrogenating the latter compound to obtain the 1-(3′,5′-disubstituted-4′-acetoxyphenyl)ethanol, and dehydrating the latter compound to form the 3,5-disubstituted-4-acetoxystyrene.

Pending Application Serial No. 226,258 filed August 2, 1988, by Aslam et al, discloses the production of 3-mono- or 3,5-disubstituted-4-acetoxystyrenes, and specifically 3,5-dibromo-4-acetoxystyrene, from the corresponding substituted 4-hydroxyacetophenone, by a process similar to that described previously in Serial No. 097,809.

Pending Application Serial No. 226,259 filed August 2, 1988, by Vicari et al, discloses the preparation of 3-substituted-4-acetoxystyrenes, e.g., 3-methyl-4-acetoxystyrene, from the corresponding 3-substituted-4-hydroxyacetophenones, using the sequence of reactions disclosed in application Serial No. 097,809.

The disclosures of the foregoing three pending patent applications are incorporated herein by reference to the extent that they disclose the production of substituted 4-acetoxystyrenes by means of processes utilizing the corresponding substituted 4-acetoxyacetophenones as intermediates.

Pending Application Serial No. 661,552 filed October 17, 1984, by Gerberich discloses the preparation of hydroquinone and related compounds utilizing a purified peracetic acid prepared from commercial

peracetic acid by acetic anhydride drying and distillation.

Pending U. S. Application Serial No. 921,702 filed October 20, 1986, by Gerberich shows the preparation of 2,6-diacetoxynaphthalene utilizing peracetic acid containing little or no water or sulfuric acid and prepared either by reacting hydrogen peroxide and glacial acetic acid in the presence of an acid ion-exchange catalyst or by subjecting a commercial peracetic acid to acetic anhydride drying and distillation.

The disclosures of the foregoing two applications related to the preparation or treatment of peracetic acid are incorporated herein by reference.

## SUMMARY OF THE INVENTION

In accordance with this invention, 4-acetoxystyrene oxide or any of certain substituted 4-acetoxystyrene oxides are produced by epoxidizing the corresponding 4-acetoxystyrene or substituted 4-acetoxystyrene with peracetic acid containing no more than a trace of a mineral acid, e.g., sulfuric acid, or water. It has been found that good yields of the 4-acetoxystyrene oxide are obtained under these conditions, whereas when commercial peracetic acid is utilized containing substantial although minor amounts of a mineral acid acid such as sulfuric acid, and water, the reaction either doesn't proceed or proceeds with the production of much lower yields of the 4-acetoxystyrene oxide.

## DESCRIPTION OF PREFERRED EMBODIMENTS

The 4-acetoxystyrene oxides of this invention are unsubstituted 4-acetoxystyrene oxide, and substituted 4-acetoxystyrene oxides wherein at least one ring carbon atom is bonded to a substituent which may be $C_1$ to $C_{10}$ alkyl or alkoxy, amino, e.g., $NH_2$, halogen, e.g., Br, Cl, or I, or nitro. Such compounds have the formula

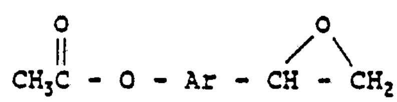

where Ar is a 1,4-phenylene group with the 1-carbon bonded to the epoxide (oxirane) group and the 4-carbon bonded to the acetoxy group, and wherein the ring carbon atoms are bonded to hydrogen or any of the substituent groups delineated previously. If more than one substituent group is present, they may be the same or different.

The prevalent method for the production of peracetic acid is the oxidation of acetic acid with hydrogen peroxide using a mineral acid, usually sulfuric acid, as an acidic catalyst resulting in a commercial peracetic acid product containing about 35-40 wt % of peracetic acid and at least about 0.5 wt %, more usually about 1 wt % of mineral acid, e.g., sulfuric acid, at least about 15 wt %, more usually about 20 wt %, of water and about 0.5 to 1.0 wt % of hydrogen peroxide.

In accordance with a preferred embodiment of the invention, the peracetic acid containing no more than a trace of a mineral acid, e.g., sulfuric acid, or water, used to react with the 4-acetoxystyrene or substituted 4-acetoxystyrene, is prepared from commercial peracetic acid by either (1) a solvent extraction with an organic solvent which dissolves acetic and peracetic acids but is immiscible with water, to separate such acids from most of the water and mineral acid, with the latter compounds forming a separate aqueous phase which may be discarded, and treatment of the solvent phase with an insoluble dehydrating agent to remove most of the residual water; or (2) a treatment with acetic anhydride to convert the residual water to acetic acid and a distillation to separate the peracetic and acetic acids from the mineral acid, e.g., sulfuric acid.

In removing all or most of the mineral acid and water from commercial peracetic acid by solvent extraction, the solvent utilized may be, for example, a halogenated, e.g., chlorinated, hydrocarbon liquid at room temperature, e.g., chloroform, methylene chloride (dichloromethane) and ethylene dichloride. The commercial peracetic acid is mixed with the solvent at a peracetic:solvent ratio of about 1:10 to 1:100 at, for example, 20 to 30 °C, preferably room temperature (25 °C), and the aqueous phase is separated and discarded. The solvent layer is dried with an insoluble dehydrating agent, e.g., magnesium sulfate, sodium

sulfate, calcium chloride or the like. Although the solvent may be removed from the peracetic and acetic acids by distillation, it is preferable in many cases to use the solution of the acids in the solvent directly in the epoxidation reaction, because of the exothermicity of such reaction.

In renoving all or most of the mineral acid and water from a commercial peracetic acid mixture by acetic anhydride addition and distillation, the mixture, after the acetic anhydride has reacted with substantially all of the residual water, is distilled at a moderate temperature, e.g., about 25 t0 50° C, under vacuum conditions, e.g., 1 to 10 mm HgA to separate the peracetic and acetic acids from the mineral acid, e.g., sulfuric acid. The resulting peracetic acid solution, which is substantially free of mineral acid and water, may then be used in the epoxidation reaction.

By the phrase "no more than a trace of" mineral acid or water is generally meant no more than about 0.05 wt % of mineral acid, and no more than about 1.0 wt % of water based on the weight of pure peracetic acid in the mixture. Preferably the mixture contains substantially no mineral acid or water.

The epoxidation of the 4-acetoxystyrene or substituted 4-acetoxystyrene, as defined previously, proceeds in accordance with Equation I:

$$CH_3\overset{\overset{O}{\|}}{C} - OAr - CH = CH_2 + CH_3COOOH \longrightarrow$$

$$CH_3\overset{\overset{O}{\|}}{C} - O - Ar - \overset{O}{\widehat{CH - CH_2}} + CH_3COOH \qquad (I)$$

where Ar has the meaning defined previously.

In carrying out the foregoing reaction, a solution of about 30 to 40 wt % of peracetic acid in acetic acid containing no more than a trace of mineral acid and water and preferably mixed with an organic solvent, is contacted with the 4-acetoxystyrene or substituted 4-acetoxystyrene, preferably mixed with the same organic solvent as the peracetic acid/acetic acid mixture. The solvent utilized may be any of the same solvents which-are suitable for the removal of mineral acid and water from commercial peracetic acid by solvent extraction as described previously, and if in fact the peracetic acid is obtained by means of such solvent extraction, the resulting mixture with solvent obtained from such extraction may be used directly for the epoxidation reaction. Moreover, if such solvent extraction is utilized in the purification of the commercial peracetic acid, the solution of 4-acetoxystyrene or substituted 4-acetoxystyrene preferably also contains a small amount of alkali metal acetate, e.g., sodium acetate, for the purpose of neutralizing any mineral acid, e.g., sulfuric acid, which remains in the peracetic acid mixture after the solvent extraction.

The epoxidation reaction is carried out at a temperature of, for example, about 0 to 70° C, for a reaction period of, for example, about 2 to 48 hours. After the reaction is complete, the reaction mixture is desirably washed with water and an aqueous solution of a mild base, e.g., sodium bicarbonate, dried over an insoluble dehydrating agent, e.g., magnesium sulfate or sodium sulfate, and concentrated under vacuum to obtain the 4-acetoxystyrene oxide or substituted 4-acetoxystyrene oxide product.

Preferably, the 4-acetoxystyrene or substituted 4-acetoxystyrene is obtained from 4-hydroxyacetophenone (4-HAP) or the corresponding substituted 4-HAP by esterifying the 4-HAP or substituted 4-HAP with an acetylating agent to obtain 4-acetoxyacetophenone (4-AAP) or the corresponding substituted 4-AAP, the latter compound is then hydrogenated or reduced with a suitable catalyst to obtain 1-(4′-acetoxyphenyl)ethanol or corresponding substituted 1-(4′-acetoxyphenyl)ethanol, and the latter compound is dehydrated to form the 4-acetoxystyrene or corresponding substituted 4-acetoxystyrene.

In carrying out the foregoing process, the 4-HAP or substituted 4-HAP is esterified with an acetylating agent to form 4-AAP or substituted 4-AAP in accordance with Equation II:

$$HOArCOCH_3 + CH_3COX \rightarrow CH_3\overset{\overset{O}{\|}}{C}OArCOCH_3 + HX \qquad (II)$$

where Ar has the meaning defined previously and X is the residue minus an acetyl group of compounds which are known acetylating agents. X may be, for example, hydroxy, acetoxy, or halide including fluoride, chloride, bromide, or iodide. Acetylating agents which may be used are for example, acetic anhydride, acetic acid, acetyl fluoride, acetyl chloride and acetyl bromide. The reaction may be carried out, for example, by contacting the 4-HAP or substituted 4-HAP with, for example, about 1 to 5 moles of the acetylating agent, e.g., acetic anhydride, per mole of 4-HAP or substituted 4-HAP, at refluxing temperature, for a period, for example, in the range of 1 to 20 hours. Excess acetylating agent, e.g., acetic anhydride, as well as the by-product of the reaction HX, e.g., acetic acid when acetic anhydride is the acetylating agent,

may be removed by vacuum distillation and the 4-AAP or substituted 4-AAP may be further purified by flash distilling under vacuum at a higher temperature.

The hydrogenation or reduction of the 4-AAP or substituted 4-AAP to obtain the desired 1-(4′-acetoxyphenyl)ethanol or substituted 1-(4′-acetoxyphenyl)ethanol (the "ethanol derivative") proceeds as shown in Equation III, where "[H]" represents the available hydrogen in hydrogen gas in the presence of a hydrogenation catalyst or in a hydrogen-containing reducing agent such as sodium borohydride or lithium aluminum hydride:

$$CH_3\overset{\overset{O}{\|}}{C}OArCOCH_3 \xrightarrow{[H]} CH_3\overset{\overset{O}{\|}}{C}OAr - \overset{\overset{CH_3}{|}}{CH} - OH \qquad (III)$$

The hydrogenation or reduction as shown in Equation III may be accomplished, for example, by contacting the 4-AAP or substituted 4-AAP as is or dissolved in an appropriate solvent with a hydrogenation catalyst in the presence of hydrogen. The solvent may be, for example, methanol, ethanol, t-butanol, aqueous alcohol, toluene, diethyl ether, tetrahydrofuran, or 1,4-dioxane, and the 4-AAP or substituted 4-AAP:solvent weight ratio may be in the range, for example of about 1:1 to 1:100, preferably about 1:2 to 1:20. The hydrogenation catalyst may be, for example, a transition metal or a reduced salt of such metal on a suitable support. Preferred transition metals are nickel, e.g., Raney nickel, and the noble metals, e.g., palladium, platinum, rhodium, iridium, ruthenium and osmium, and some suitable supports are, for example, carbon, alumina, silica, and polymeric resins. The metal concentration on the support in weight ratio of metal:support may be in the range, for example, of about 1:100 to 1:2, preferably about 1:50 to 1:10, and the weight ratio of catalyst system:4-AAP or substituted 4-AAP is, for example, in the range of about 1:500 to 1:2, preferably about 1:30 to 1:5. In carrying out the reaction, the hydrogen pressure may be in the range, for example, of about 10 to 1200 psig, preferably about 50 to 300 psig; the reaction temperature may be in the range, for example, of about 10 to 150 °C, preferably about 20 to 80 °C; and the reaction time may be in the range, for example, of about 0.25 to 10.0 hours, preferably about 1.0 to 4.0 hours. Under some conditions, the addition of a base or passivation of the reactor with base, may be desirable to prevent hydrogenolysis.

Alternative to the hydrogenation reaction as described, the reduction reaction shown in Equation III may be accomplished, for example, by slowly adding to a cooled solution of 4-AAP or substituted 4-AAP in an alcohol, e.g., methanol, ethanol, or t-butanol, or an ether such as tetrahydrofuran or diethyl ether, a reducing agent containing available hydrogen, e.g., sodium or potassium borohydride or lithium aluminum hydride. The solution nay then be warmed to room temperature and heated at reflux, e.g., for a period of about 0.5 to 3.0 hours. Water may then be added and the product extracted with a water insoluble organic solvent, e.g., ethyl acetate. The solution is decanted, dried with a dehydrating agent such as magnesium sulfate and concentrated in a rotary evaporator to yield the ethanol derivative product.

Reduction with a hydrogen containing reducing agent rather than hydrogenation with a hydrogenation catalyst is particularly useful when a ring substituent is present, i.e., halogen, which has a tendency to react with hydrogen and be removed from the ring.

The ethanol derivative is then dehydrated to form the 4-acetoxystyrene or corresponding substituted 4-acetoxystyrene, in accordance with Equation IV:

$$CH_3\overset{\overset{O}{\|}}{C}OAr - \overset{\overset{CH_3}{|}}{CH} - OH \xrightarrow[\text{dehyd. agent}]{\text{heat}} CH_3\overset{\overset{O}{\|}}{C}OAr - CH = CH_2 + H_2O \qquad (IV)$$

The dehydration reaction is carried out by heating the ethanol derivative under vacuum in the presence of a polymerization inhibitor and a dehydrating agent. Dehydrating agents which can be used include, for example, $KHSO_4$, $CuSO_4$, $CuCl_2$ and $Al_2O_3$. Polymerization inhibitors which can be used include, for example, t-butyl catechol, hydroquinone, tetrachloroquinone and di-t-butyl-p-cresol. The dehyrating agent may be present in an amount, for example, of from about 0.25 to about 5.0 percent weight of the ethanol derivative, and the polymerization inhibitor may be present, for example, in an amount of from about 1% to about 5% based on the weight of the ethanol derivative. The reaction vessel is heated to from about 160° to about 210 °C, preferably 168 °C to about 190 °C under vacuum conditions, e.g., from about 0.01 to about 30 mm Hg for a reaction period of, for example, about 0.25 to 3.0 hours to obtain the 4-acetoxystyrene. The

product may then be purified by conventional means, e.g., distillatior solvent extraction, selective adsorption of color bodies, etc.

The following examples further illustrate the invention:

## EXAMPLE 1

This example illustrates the preparation of 4-acetoxystyrene ox de from 4-acetoxystyrene under the invention, the latter compound having been prepared from 4-hydro /acetophenone (4-HAP) as an intermediate.

A solution of 136.2g (1.0 mol) of 4-hydroxyacetophenone and 40C ml of acetic anhydride was heated at reflux for 3 h under a nitrogen atmosphere. The acetic acid and acet anhydride was distilled overhead in vacuo (30-41° C, 2.6 mm Hg). The remaining oil was then distilled vacuo (132-134° C, 2.0 mm Hg) to yield 169.7g (95.2%) of white crystals identified as 4 -acetoxyacetoph none.

4-Acetoxyacetophenone (100.0 g, 0.56 mol) was hydrogenated a Fluidtron Reactor with 5% Pd/C (3.94 g) at 100 psig. The hydrogenation was carried out at 60° C for 5.25 hours. The reactor was depressurized and the catalyst removed via filtration to afford 1-(4′-ac oxyphenyl)ethanol as an oil (93.6 g).

1-(4′-Acetoxyphenyl)ethanol (250.0 g, 1.39 mol), $KHSO_4$ (0.25 ) and t-butyl catechol (2.5 g) were mixed in a flask equipped with a fractional distillation apparatus and attached to a vacuum pump. Dehydration was conducted at 141-177° C under vacuum (10.0 mm g). The product was collected as an oil along with some water. The water layer was separated and the c ganic layer collected (174.24 g). The yield of 4-acetoxystyrene was determined to be 69.7%.

Commercial peracetic acid produced by the oxidation of aceti acid with hydrogen peroxide using sulfuric acid as catalyst, and containing about 35 wt % of peracetic a d, about 19 wt % of water, about 0.6 wt % of hydrogen peroxide, about 1 wt % of sulfuric acid, and the r mainder acetic acid was mixed in an amount of 51 g with chloroform (500 ml) and transferred to a separ ory funnel. The upper aqueous layer was discarded and the chloroform layer was dried over magnesium ulfate and filtered to yield a solution containing about 37 wt % of peracetic acid, about 0.6 wt % of hydroc n peroxide, and the remainder acetic acid. No more than a trace of sulfuric acid or water is detectable. T e solution was added dropwise to an ice cold solution of 4-acetoxystyrene (24.3 g, 0.15 mol) in chlorofor (150 ml) containing sodium acetate (5.0 g). After the addition of peracetic acid solution, the reactic mixture was warmed up to room temperature and stirred overnight. The reaction mixture was washed /ith water (3 x 200 ml) and saturated sodium bicarbonate solution (3 X 200 ml), dried over anhydrous agnesium sulfate, filtered, and concentrated in-vacuo to afford an oil characterised by NMR analysis as 4-acetoxystyrene oxide (27.0 g, 98% yield).

The foregoing commercial peracetic acid (19.0 g) without any pu ication was added dropwise to an ice cold solution of 4-acetoxystyrene (8.1 g, 0.05 mol) in chloroform (50 r ). After the addition of peracetic acid, the reaction mixture was warmed to room temperature and stirred ov night. Workup as above gave a thick oil (3.9 g). NMR analysis revealed that 4-acetoxystyrene oxide as not made under these reaction conditions.

## EXAMPLE 2

This example illustrates the preparation of 3,5-dimethyl-4-acet ystyrene oxide from 3,5-dimethyl-4-acetoxystyrene by means of the process of this invention, the latter ompound having been prepared by a process utilizing 3,5-dimethyl-4-hydroxyacetophenone as an intermec te.

3,5-Dimethyl-4-hydroxyacetophenone (47.4 g) prepared as des bed in U. S. Application Serial No. 097,809 previously cited, was esterified by refluxing with 4 mols c acetic anhydride for 19 hours. After removal of acetic acid and acetic anhydride by vacuum distillation, th 3,5-dimethyl-4-acetoxyacetophenone was flash distilled to yield 47.7g of a slightly yellow liquid. There vere then mixed 0.1 mol of the 3,5-dimethyl-4-acetoxyacetophenone, 1.2g of 5% Pd/C as catalyst anc 00 ml ethanol in an autoclave. The autoclave was charged with hydrogen gas at a pressure of 215-220 ig and the reaction was run for about 2-1/2 hours at a temperature of about 25-30° C. Additional catalyst w s added as needed. The catalyst was then removed and the ethanol evaporated to yield 21.5g of a color ss oil which was 1-(3′,5′-dimethyl-4′-acetoxyphenyl) ethanol.

There were added to a flask 0.168 mol of the 1-(3′,5′-dimethy 4′-acetoxyphenyl)ethanol with 0.35 g $KHSO_4$ and 0.5g t-butyl catechol. The flask was heated to 185 to 1 ° C at 1.5 to 2.0 mm Hg. A colorless

liquid was distilled. After the addition of 0.15 g of t-butyl catechol, and redistillation, 26.1 g of 3,5-dimethyl-4-acetoxystyrene were produced. The compound had a boiling point of 90-91 °C at 0.5 mm of Hg and the yield was 81.8%.

Commercial peracetic acid (10.9 g), as described in Example 1, was purified by extraction with chloroform and drying with anhydrous magnesium sulfate as described in the same example. The dried peracetic acid in chloroform solution was filtered and added to a solution of 3,5-dimethyl-4-acetoxystyrene (4.0 g), sodium acetate (1.0 g) and chloroform (50 ml). The reaction mixture was stirred at room temperature for 24 h and washed with saturated sodium bicarbonate solution and water. The chloroform layer was separated, dried and concentrated to afford a white oil which solidified upon cooling (3.6 g, yield). Recrystallization with $CCl_4$/hexane afforded white crystals; m.p. 42-46 °C. NMR analysis indicated the crystals to be 3,5-dimethyl-4-acetoxystyrene oxide.

EXAMPLE 3

This example illustrates the preparation of 3,5-dibromo-4-acetoxystyrene oxide from 3,5-dibromo-4-acetoxystyrene under the invention, the latter compound having been prepared from 3,5-dibromo-4-hydroxyacetophenone as an intermediate.

3,5-Dibromo-4-hydroxyacetophenone (154.0 g, 0.523 mol) prepared as described in U. S. Application Serial No. 226,258 previously cited, acetic anhydride (100.0 ml) and sodium acetate (1.0 g) were placed in a one liter flask and heated to reflux overnight. Unreacted acetic anhydride and acetic acid were removed via distillation at 0.1-0.25 mm Hg. A solid material was left in the flask. Recrystallization with isopropyl alcohol (110 ml) afforded a light yellow crystalline solid. The crystals were dried in a vacuum oven to yield 161.8 g of 3,5-dibromo-4-acetoxyacetophenone.

3,5-Dibromo-4-acetoxyacetophenone (35.2 g, 0.1 mol) was suspended in a flask in absolute alcohol (100 ml) The flask was cooled in ice and sodium borohydride (1.9, 0.05 mol) was added slowly to the cooled reaction mixture which was stirred at 0 °C for two hours. Water (300 ml) was added and the product was extracted with ethyl acetate (300 ml). The organic layer was separated, dried over magnesium sulfate and concentrated on the rotary evaporator to yield 30.7g of 1-(3′,5′-dibromo-4′-acetoxyphenyl) ethanol.

1-(3′,5′-Dibromo-4′-acetoxyphenyl)ethanol (104.86 g, 0.296 mol) $KHSO_4$ (1.0 g) and t-butyl catechol (3.34 g) were mixed in a flask equipped with a fractional distillation apparatus and attached to a vacuum pump. Dehydration was conducted at 168-187 °C at a vacuum pressure of 0.05-0.10 mm Hg. The product was distilled at 116-134 °C to yield an oily solid (61.82 g) which was indicated by analysis to be 3,5-dibromo-4-acetoxystyrene.

Purified peracetic acid prepared from commercial acid by acetic anhydride drying and distillation (37%) (3.2 g) was mixed with chloroform (6.0 ml) and added to a solution of 3,5-dibromo-4-acetoxystyrene (2.0 g), sodium acetate (0.3 g), and chloroform (12.5 ml). The reaction mixture was heated to 50 °C and stirred for 18 h. The reaction mixture was washed with water (3 X 100 ml) saturated sodium bicarbonate solution (3 X 100 ml) and water (2 X 100 ml), dried over anhydrous magnesium sulfate, filtered and concentrated with the rotary evaporator and the vacuum pump to afford a yellow oil (1.8 g, 90%). NMR analysis revealed that the reaction went to 94% completion. GC analysis revealed that the product was 87% pure 3,5-dibromo-4-acetoxystyrene oxide. Recrystallization with $CCl_4$/hexane afforded a white solid; m.p. 75-76 °C.

Results of the epoxidation reactions similar to those of Examples 1 and 2 could also be obtained using peracetic acid purified from the same commercial peracetic acid described in Example 1 but using acetic anhydride drying and distillation, as practiced in Example 3 and described in U. S. Applications Serial Nos. 661,552 and 921,702, previously cited.

**Claims**

1. A process for the production of a 4-acetoxystyrene oxide or a substituted 4-acetoxystyrene oxide wherein at least one of the ring hydrogen atoms is substituted by a $C_1$ to $C_{10}$ alkyl, or alkoxy, amino, halogen or nitro, characterised in that the correponding 4-acetoxystyrene or substituted 4-acetoxystyrene is epoxidized with peracetic acid containing no more than a trace of mineral acid or water.

2. The process of Claim 1 wherein 4-acetoxystyrene oxide is produced from 4-acetoxystyrene.

3. The process of Claim 1 wherein 3,5-dimethyl-4-acetoxystyrene oxide is produced from 3,5-dimethyl-4-acetoxystyrene.

4. The process of Claim 1 wherein 3,5-dibromo-4-acetoxystyrene oxide is produced from 3,5-dibromo-4-acetoxystyrene.

5. The process of any of Claims 1-4 wherein said peracetic acid is produced from commercial peracetic acid containing at least 0.5 wt % of sulfuric acid and at least 15 wt % of water by subjecting said commercial peracetic acid to extraction with a water-immiscible organic solvent which dissolves peracetic and acetic acids, discarding the aqueous phase and drying the solvent phase with an insoluble dehydrating agent.

6. The process of Claim 5 wherein said solvent is chloroform.

7. The process of any of Claims 1-4 wherein said peracetic acid is produced from commercial peracetic acid containing at least 0.5 wt % of sulfuric acid and at least 15 wt % of water by treating said commercial peracetic acid with sufficient acetic anhydride to convert the water present to acetic acid and distilling the resulting mixture to separate the peracetic and acetic acids from the sulfuric acid.

8. The process of any of Claims 1-7 which comprises acetylating the corresponding 4-hydroxyacetophenone or substituted 4-hydroxyacetophenone to produce the corresponding 4-acetoxyacetophenone or substituted 4-acetoxyacetophenone, subjecting the latter compound to hydrogenation with a hydrogenation catalyst or reduction with a hydrogen-containing reducing agent to obtain the corresponding 1-(4′-acetoxyphenyl)ethanol or substituted 1-(4′-acetoxyphenyl)ethanol, and dehydrating the latter compound to obtain the corresponding 4-acetocystyrene or substituted 4-acetoxystyrene.

9. 3,5-dimethyl-4-acetoxystyrene oxide.

10. 3,5-dibromo-4-acetoxystyrene oxide.

European Patent Office

# EUROPEAN SEARCH REPORT

Application number
EP 89308121.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | CHEMICAL ABSTRACTS, vol. 99, no. 14, October 3, 1983, Columbus, Ohio, USA ASAHI GLASS CO. "Semi-permeable membranes" page 56, column 1, Abstract-no. 106 524h & Jpn. Kokai Tokkyo Koho JP 58 92 406 (83 92 406) -- | 1,9,10 | C 07 D 301/14<br>C 07 D 303/16 |
| A | CHEMICAL ABSTRACTS, vol. 97, no. 20, November 15, 1982, Columbus, Ohio, USA KONDO, T. et al. "Methyl 5-epoxyacetylsulicylate. Preparation and copolymerization" page 2, column 1, Abstract-no. 163 534u & Polym. Bull. (Berlin) 1982 7(11-12), 513-20 -- | 1,9,10 | |
| | | | TECHNICAL FIELDS SEARCHED (Int. Cl.4) |
| A | CHEMICAL ABSTRACTS, vol. 98, no. 4, January 24, 1983, Columbus, Ohio, USA DIMMEL, D.R. et al. "Synthesis of lignin model dimers by novel techniques" page 85, column 1, Abstract-no. 18 276c & J. Wood. Chem. Technol: 1982, 2(3), 297-315 ---- | 1,9,10 | C 07 D 301/00<br>C 07 D 303/00 |

The present search report has been drawn up for all claims

| Place of search | Date of completion of the search | Examiner |
|---|---|---|
| VIENNA | 13-10-1989 | BRUS |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82